# EUROPÄISCHE PATENTSCHRIFT

(11) **EP 0 596 145 B1**
(45) Veröffentlichungstag und Bekanntmachung des Hinweises auf die Patenterteilung: **08.05.1996**
(21) Anmeldenummer: 92118671.4
(22) Anmeldetag: 31.10.1992
(51) Int. Cl.: A61F 2/06

(54) **Anordnung zum Implantieren von selbstexpandierenden Endoprothesen**
Disposition for implanting a self-expanding endoprothesis
Disposition d'introduction d'une endoprothése autoexpansible

(43) Veröffentlichungstag der Anmeldung: 11.05.1994
(73) Patentinhaber: SCHNEIDER (EUROPE) AG, CH-8180 Bülach (CH)
(72) Erfinder: Braunschweiler, Reto, Ch-8405 Winterthur (CH); Bachmann, Michel, CH-1032 Romanel (CH)
(74) Vertreter: Rottmann, Maximilian R.

(56) Entgegenhaltungen:
- EP-A- 0 408 245
- WO-A-87/04935
- US-A- 4 665 918

## Beschreibung

Die Erfindung betrifft eine Anordnung mit einer selbstexpanierenden Endoprothese und einer Vorrichtung zu deren Implantation nach dem Oberbegriff des Patentanspruchs 1.

Endoprothesen der gattungsgemässen Art können beispielsweise in Venen, Gallen- oder Harnwegen zur Erhaltung der Durchgängigkeit eingebracht werden. Auch werden sie zur Verhütung von Rezidivstenosen im Sinne eines elastischen Recoilings oder einer narbigen Konstriktion nach durchgeführter Ballondilatation von Arterien eingesetzt.

Bei den meisten bekannten Anordnungen ist die achsiale Länge der Endoprothese im zusammengefalteten Zustand wesentlich grösser als im aufgeweiteten Zustand. Daher ist die Positionierung in Gefässen und dergleichen relativ schwierig, da die genaue Länge und Lage der Endoprothese erst nach deren Entfaltung ersichtlich ist. Bei den meisten bekannten Anordnungen können die Endoprothesen durch eine Relativbewegung zwischen dem rohrförmigen Körper und dem Kernelement freigesetzt werden.

Allen heute auf dem Markt erhältlichen Anordnungen ist jedoch gemeinsam, dass die Endoprothese, sobald sie teilweise freigesetzt ist, nicht mehr zurückgefaltet werden kann. Das bedeutet, dass, sobald die Endoprothese auch nur ein kleines Stück freigegeben ist, die Vorrichtung nur noch aus dem Körper entfernt werden kann, indem die Endoprothese ganz freigesetzt und im Körper zurückgelassen wird. Sobald die Endoprothese teilweise freigesetzt ist, kann sie ausserdem nur noch proximal gezogen, jedoch nicht gestossen werden, da die Endoprothese beim Stossen mit ihrem distalen und aufgeweiteten Ende das Gefäss, in das sie eingebracht werden soll, unweigerlich verletzen würde. Auch die Form der Endoprothese würde durch diese Krafteinwirkung beeinträchtigt; sie würde gestaucht werden und Ihre Stützelemente würden knicken.

Wenn beim Freigeben und Entfalten der Endoprothese festgestellt wird, dass die Endlage nicht mit der gewünschten Position in Übereinstimmung kommen wird, sollte somit die Möglichkeit bestehen, die Endoprothese nach dem teilweisen Freigeben auch wieder in die Vorrichtung hineinzuziehen, damit sie im zusammengefalteten Zustand entsprechend neu positioniert werden kann.

In der EP-A-0408245 ist eine solche, als Instrument zum Anbringen eines selbst-expandierenden Aufweitimplantats bezeichnete Anordnung umschrieben.

Diese Anordnung besteht im wesentlichen aus einer äusseren Hülse, in welcher axial ein hohler Kern liegt. Am distalen Ende weist der Kern einen stufig erhöhten Durchmesser auf, wobei das distale Ende des Kerns auf das distale Ende der Hülse trifft. Um den Kern ist an seinem distalen Ende ein Greifglied angeordnet, das lösbar ein selbstexpandierendes Aufweitimplantat festhält. Um eine Reibschlussverbindung zwischen dem Kern bzw. dem Greifglied und der Aufweit-Prothese herzustellen, besteht das Greifglied aus einem Material mit hoher Reibung. Eine weitere Ausführungsform sieht zudem noch eine Beschichtung des Greifgliedes mit einem Mittel zum lösbaren Ankleben des Aufweitimplantats vor.

Nachteilig bei dieser Anordnung ist, dass die äussere Hülse das Aufweitimplantat eng umschliessen muss, damit wie vorgeschlagen eine Reibschlussverbindung zwischen dem Greifglied und dem selbstexpandierenden Aufweitimplantat gewährleistet ist. Dadurch entsteht bei einer Relativverschiebung zwischen dem Kern und der äusseren Hülse, natürlich auch zwischen dem Aufweitimplantat und der Innenwand der äusseren Hülse, eine hohe Reibung, so dass dadurch die aufzuwendenden Kräfte zum Freigeben des Aufweitimplantats bzw. zum Zurückziehen desselben gross sind. Im weiteren besteht die Gefahr, dass durch die relativ hohen aufzuwendenden Kräfte die Zuverlässigkeit in Mitleidenschaft gezogen respektive eine genaue Positionierung entsprechend erschwert wird, weil zu der Reibung zwischen Aufweitimplantat und äusserer Hülse noch die Reibung des Kerns an der äusseren Hülse auf der ganzen restlichen Länge hinzukommt. Ausserdem muss ein hoher Aufwand getrieben werden, um durch Gestaltung der entsprechenden Reibbeiwerte sicherzustellen, dass die Relativbewegung tatsächlich wie beabsichtigt zwischen Aufweitimplantat und äusserer Hülse stattfindet, obwohl dort die Normalkraft auf die Reibflächen um den Betrag der Expansionskraft des Aufweitimplantats höher liegt als die Normalkraft zwischen Kern und Aufweitimplantat.

Entscheidend ist, dass bei dieser Anordnung zur Erzielung einer ganz bestimmten Reibung zwischen Kern und Aufweitimplantat auch eine ebenfalls entsprechend genau bestimmte Normalkraft erforderlich ist. Die Grösse der Normalkraft ist in diesem Fall festgelegt durch die Elastizität der äusseren Hülse, die Elastizität des Aufweitimplantats und die Elastizität des Kerns auf der einen Seite und durch die Abmessungen des Innendurchmessers der äusseren Hülse, die Abmessung der Wandstärke des Aufweitimplantats und die Abmessung des Aussendurchmessers des Kerns auf der anderen Seite. Ausserdem bestimmt noch die Expansionskraft des Aufweitimplantats die Grösse der Normalkraft; sie wirkt der Normalkraft entgegen. Die notwendige Normalkraft muss, um eine bestimmte Reibkraft zu erzeugen, recht genau eingehalten werden; andererseits reagiert die Normalkraft jedoch sehr empfindlich auf die angegebenen Bestimmungsfaktoren. Eine Serienfertigung dieser Anordnung bereitet daher grosse Schwierigkeiten.

Die in der erwähnten EP im weiteren vorgeschlagene Beschichtung des Greifglieds mit einem Mittel zum lösbaren Ankleben des Aufweitimplantats bringt die Gefahr mit sich, dass sich das Aufweitimplantat, insbesondere bei längerer Lagerung, nach dem Zurückziehen der äusseren Hülle nicht mehr von selbst aufweitet, da es eine zu geringe Öffnungskraft besitzt, oder dass sich die Klebeschicht mit der Zeit chemisch verändert und dadurch die angestrebte Reibschlussverbindung nicht mehr gewährleistet ist. Schwierigkeiten bereitet auch das Anbringen der Klebeschicht, es muss auf jeden Fall verhindert werden, dass die Klebeschicht zwischen Elemente des Aufweitimplantats gelangt, die sich beim Expandieren des Implantats gegeneinander bewegen müssen. Wieder lösbare Klebemittel erzielen ihre Eigenschaften dadurch, dass sie ständig gewisse Fliesseigenschaften bewahren; sie härten nicht aus. Es besteht damit die Gefahr, dass ursprünglich richtig angebrachte Klebemittel im Laufe der Lagerzeit der Anordnung zu Fliessen beginnen und das Aufweitimplantat verkleben.

Eine andere Ausführungsform der erwähnten EP sieht vor, die Oberfläche des Greifgliedes mit einer aufgerauhten Oberfläche zu versehen. Bei einer aufgerauhten Oberfläche besteht jedoch die Gefahr, dass das Aufweitimplantat verformt wird und dadurch in seiner Form, insbesondere im aufgeweiteten Zustand, beeinträchtigt wird.

Generell ist festzuhalten, dass durch Ausführungsformen, wie sie in der erwähnten EP vorgeschlagen werden, eine sichere Funktionsweise nicht in allen Fällen erreicht werden kann, da bei der Herstellung der Aufweitimplantate und insbesondere auch bei der Art des Zusammenpressens mit grossen Toleranzen bezüglich der Masshaltigkeit und der Aufweitkraft gerechnet werden muss.

Es ist daher die Aufgabe der Erfindung, eine einfache Anordnung zum Implantieren und Zurückfalten von Endoprothesen zu schaffen, bei welcher gegenüber Anordnungen, die keine Zurückfaltung der Endoprothese erlauben, die Bedienungskraft nicht erhöht und die freie und unverformte Entfaltung der Endoprothese nicht beeinträchtigt ist, welche leicht und ohne grossen Aufwand herstellbar ist, welche eine sichere Funktionsweise auch nach langer Lagerung gewährleistet, und bei welcher auch gewisse unvermeidliche Herstellungstoleranzen der Endoprothesen keinen nachteiligen Einfluss auf eine sichere Funktionsweise haben, insbesondere beim Freigeben und beim Zurückfalten der Endoprothese.

Eine weitere Aufgabe der Erfindung besteht darin, ein Verfahren zum Anbringen einer Prägung an einer solchen Anordnung zu schaffen, durch welches die Prägung auf einfache Art und Weise angebracht werden kann.

Diese Aufgaben werden durch die im kennzeichnenden Teil des Anspruchs 1 beschriebene Anordnung sowie durch das im kennzeichenenden Teil des Anspruchs 16 beschriebene Verfahren gelöst.

Eine solche Ausgestaltung der Vorrichtung, bei der das Kernelement ein mit der Struktur der inneren Oberfläche der Endoprothese korrespondierend auf einem Teil seiner Oberfläche angebrachtes Relief aufweist, gewährleistet, dass einerseits die aufzuwendenden Kräfte zum Bewegen des Kernelements relativ zum äusseren Körper gering gehalten werden können, und dass andererseits ein sicheres radiales Ablösen der selbstexpandierenden Endoprothese vom Kernelement auch nach langer Lagerung noch gewährleistet ist. Somit wird durch eine solche Ausgestaltung eine sichere und zuverlässige Funktionsweise erreicht. Auch kann eine solche Vorrichtung leicht und ohne grossen Aufwand hergestellt werden.

Eine bevorzugte Ausführungsform der Erfindung sieht vor, dass das auf dem Kernelement angebrachte Relief individuell der Struktur der inneren Oberfläche der Endoprothese angepasst ist. Durch eine solche Ausgestaltung wird sichergestellt, dass eine optimale Formschlussverbindung zwischen dem Kernelement und der Endoprothese erreicht und daher eine höchstmögliche Funktionssicherheit gewährleistet wird. Die einzelnen Formschlusseingriffsstellen sind in diesem Fall in ihrer Lage, Anordnung und Ausbildung einzeln aufeinander abgestimmt.

Bei einem bevorzugten Verfahren ist vorgesehen, die individuell in der Anordnung zum Einsatz kommende Endoprothese, zum Ausbilden des Reliefs einzusetzen. Ein solches Verfahren hat den Vorteil, dass selbst grosse Toleranzen bezüglich der Masshaltigkeit der Endoprothese sich nicht nachteilig auf die Funktionssicherheit der Anordnung auswirken.

Im folgenden werden zwei Ausführungsbeispiele der Erfindung anhand von Zeichnungen näher erläutert. In den Zeichnungen zeigen:
- Fig.1: Eine Anordnung in teilweise geschnittener Ansicht mit separat dargestellter und zusammengefalteter Endoprothese;
- Fig.2: Die Anordnung in teilweise geschnittener Ansicht mit eingeschlossener Endoprothese;
- Fig.3: Einen vergrösserten Querschnitt durch eine erste Ausführungsform der Anordnung entlang der Linie A-A in Fig.2;
- Fig.3a: Einen vergrösserten Querschnitt durch eine zweite Ausführungsform der Anordnung entlang der Linie A-A in Fig.2; und
- Fig.4: Die Anordnung in teilweise geschnittener Ansicht mit teilweise entfalteter Endoprothese.

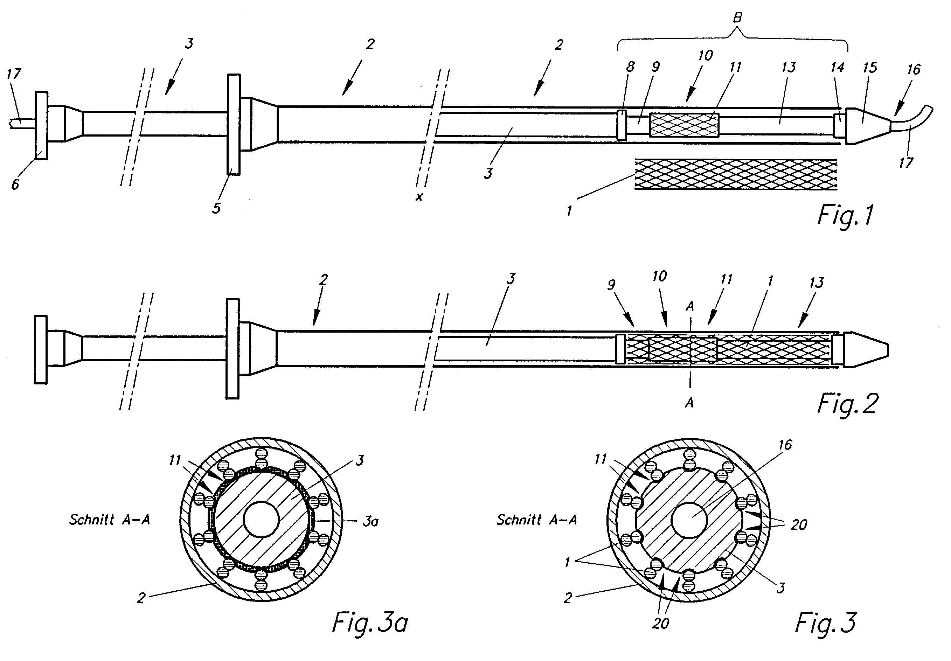

Die in der Figur 1 dargestellte Anordnung zum Implantieren von Endoprothesen weist im wesentlichen ein eine Vorrichtung mit einem äusseren rohrförmigen und flexiblen Körper 2 und einem langgestreckten, flexiblen Kernelement 3. sowie eine Endoprothese 1 auf.

Der äussere rohrförmige Körper 2 ist von der Bruchlinie x her bis zu seinem distalen Ende in Längsrichtung aufgeschnitten dargestellt. Am proximalen Ende verfügt der rohrförmige Körper 2 über einen Handgriff 5. Das langgestreckte Kernelement 3 ist im rohrförmigen Körper 2 angeordnet, wobei das Kernelement 3 länger als der rohrförmige Körper 2 ausgebildet ist und am proximalen Ende ebenfalls einen Handgriff 6 besitzt. Gegen das distale Ende hin weist das Kernelement 3 einen Bereich B auf, welcher der Aufnahme der Endoprothese 1 dient. Dieser Bereich B weist am proximalen Ende eine aus strahlungsopakem Material bestehende Schulter 8 auf. Distal daran anschliessend folgt ein im Durchmesser reduzierter Abschnitt 9. Auf den Abschnitt 9 folgt ein Bereich 10, in welchen ein Relief in Form einer Prägung 11 eingelassen ist und der einen etwas grösseren Durchmesser als der vorhergehende Abschnitt 9 aufweist. Das Relief bzw. die Prägung 11 besitzt eine mit der Struktur der inneren Oberfläche der zusammengefalteten Endoprothese 1 korrespondierende Form. An den Bereich 10 schliesst sich ein Abschnitt 13 an, welcher in etwa den gleichen Durchmesser wie der Abschnitt 9 aufweist. Darauf folgt ein wiederum aus strahlunsopakem Material bestehender Ring 14, welcher gegenüber dem Abschnitt 13 im Durchmesser etwas grösser ausgebildet ist. Am distalen Ende schliesslich weist das Kernelement 3 eine kegelstumpfförmig ausgebildete Spitze 15 auf.

Über die ganze Länge des Kernelements 3 erstreckt sich ein Lumen 16, in welches ein Führungsdraht 17 eingeschoben werden kann. Der besseren Übersichtlichkeit wegen ist die Endoprothese 1 in dieser Darstellung ausserhalb der Vorrichtung und zusammengefaltet dargestellt. Auf diese Art ist die Korrelation zwischen der Form der Prägung 11 und der zusammengefalteten Endoprothese 1 ersichtlich. Aus dieser Darstellung ist auch deutlich zu sehen, dass die Länge des mit der Prägung 11 versehenen Bereichs 10 weniger beträgt als die Länge der Endoprothese, vorzugsweise etwa 10-50% davon. Durch eine solche Länge der Prägung 11 wird die Flexibilität der Vorrichtung im Bereich B der Endoprothese 1, durch die Materialanhäufung und den Formschluss im Prägungsbereich, möglichst wenig beeinträchtigt. Andererseits ist doch eine sichere Formschlussverbindung zwischen der eingeschlossenen Endoprothese 1 und dem Kernelement 3 gewährleistet.

Aus der Fig. 2 ist die Anordnung im gebrauchsfertigen Zustand zu sehen. Die Endoprothese 1 ist hierbei zwischen dem Kernelement 3 und dem äusseren rohrförmigen Körper 2 zusammengefaltet eingeschlossen. Die Endoprothese 1 steht dabei mit ihrer inneren Oberfläche mit dem Kernelement 3 über die im Bereich 10 angebrachte Prägung 11 im Eingriff. Die Prägung 11 korrespondiert mit der Struktur der inneren Oberfläche der Endoprothese 1, so dass zwischen dem Bereich 10 des Kernelements 3 und der Endoprothese 1 eine Vielzahl von Formschlusseingriffsstellen gebildet ist. Da die einzelnen Fäden einer Lage der Endoprothese 1 beim Zusammenfalten derselben auseinanderstreben, könnten sich im Bereich des proximalen und distalen Endes der Endoprothese 1 Differenzen in der Lage der Fäden zu der Prägung 11 ergeben. Aus diesem Grund ist es vorteilhaft, zwischen dem die Prägung 11 aufweisenden Bereich 10 und den Enden der Endoprothese 1 Abschnitte 9, 13 auszubilden, welche einen geringeren Durchmesser als der Bereich 10 aufweisen.

In der Fig. 3 ist ein erstes Ausführungsbeispiel der Anordnung in einem vergrösserten Querschnitt entlang der Linie A-A in Fig.2 zu sehen. Die Endoprothese 1 ist zwischen dem äusseren rohrförmigen Körper 2 und dem Kernelement 3 eingeschlossen. Der Abstand zwischen dem Aussendurchmesser des Kernelements 3 und dem Innendurchmesser des äusseren rohrförmigen Körpers 2 ist dabei so gewählt, dass die Endoprothese 1 mit ihrer inneren Lage in die durch die Prägung 11 entstehenden Ausnehmungen gedrückt wird. Die Tiefe der Prägung 11 entspricht in etwa 50% der Dicke der inneren Lage des Halbzeugs der Endoprothese 1. Als Halbzeug zur Herstellung der Endoprothese 1 ist in diesem Fall ein dünner Draht eingesetzt. Die gleichen Verhältnissse treten jedoch auf, wenn die Endoprothese 1 beispielsweise aus dünnem Blech ausgestanzt ist oder aus Streifen hergestellt ist. Die angegebene Tiefe der Prägung 11 gewährleistet durch eine Vielzahl von Formschlusseingriffsstellen 20 eine gute Formschlussverbindung zwischen dem Kernelement 3 und der Endoprothese 1 und garantiert ausserdem auch ein sicheres radiales Lösen und Entfalten der freigesetzten Endoprothese 1, weil eine relative Bewegung der Halbzeuglagen zueinander durch die Formschlussverbindung nicht beeinträchtigt wird.

In der Fig. 3a ist ein zweites Ausführungsbeispiel der Anordnung in einem vergrösserten Querschnitt entlang der Linie A-A in Fig.2 zu sehen. Das Kernelement 3 weist in diesem Beispiel eine Beschichtung bzw. Ummantelung 3a auf. Der Vorteil einer solchen Beschichtung bzw. Ummantelung 3a ist, dass diese andere Eigenschaften als das Kernelement 3 aufweisen kann. Durch diese Ummantelung bzw. Beschichtung 3a lässt sich auch leicht die in diesem Bereich notwendige Durchmesserveränderung erzielen. So kann beispielsweise die thermoplastische Verformbarkeit des Kernelements 3 geringer als diejenige der Beschichtung oder Ummantelung 3a sein. Durch die Wandstärke der Beschichtung bzw. Ummantelung 3a kann somit die Tiefe der auszubildenden Prägung 11 einfach beeinflusst werden. Die Ummantelung 3a kann z.B. besonders einfach durch Aufschrumpfen eines Schrumpfschlauchs hergestellt werden.

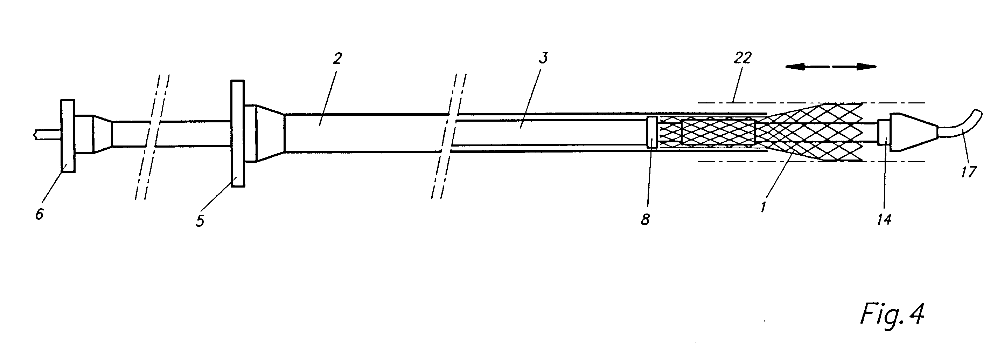

Anhand der Fig. 4 wird die Funktionsweise der Anordnung erläutert. Die Endoprothese 1 wird in bekannter Weise, im zusammengefalteten Zustand (Fig. 2), mittels der Vorrichtung in einen nur schematisch angedeuteten Körperkanal 22 eingeführt und soweit vorgeschoben, bis der distale Endbereich B der Vorrichtung mit der eingeschlossenen Endoprothese 1 an der vorgesehenen Stelle im Körperkanal 22 positioniert ist. Das Vorschieben der zusammengefalteten Endoprothese 1 im Körperkanal 22 wird dabei durch bekannte Verfahren wie etwa die Fluoroskopie überwacht. Durch die im Bereich der beiden Enden der eingeschlossenen Endoprothese 1 am Kernelement 3 angebrachten, strahlungsopaken Ringe 8, 14, ist die Lage der Endoprothese 1 gut ersichtlich.

Sobald die Endoprothese 1 die voraussichtliche Endposition eingenommen hat, wird das Kernelement 3 fixiert und die Endoprothese 1 langsam durch Zurückziehen des äusseren rohrförmigen Körpers 2 freigesetzt. Dazu dienen die beiden Handgriffe 5 und 6. Da die Endoprothese 1 selbstexpandierend ist, beginnt sich diese auf dem freigesetzten Teil zu entfalten und an die Innenwand des Körperkanals 22 anzulegen. Durch das Entfalten wird die Länge der Endoprothese 1 entsprechend verkürzt. Da zwischen der Verkürzung der Endoprothese 1 und dem Innendurchmesser des Körperkanals bzw. dem Durchmesser der Endoprothese 1 im eingesetzten, entfalteten Zustand ein direkter Zusammenhang besteht, das endgültige Aufweitmass aber nicht exakt bekannt ist, kann die endgültige Lage der entfalteten Endoprothese 1 nicht mit Sicherheit vorausbestimmt werden. Somit muss die Position der Endoprothese 1 auch während der Entfaltung überwacht werden. Zeigt sich dabei, dass deren Position korrekt ist, so kann die Endoprothese 1, durch Zurückziehen des rohrförmigen Körpers 2 bis in den Bereich der Schulter 8 des Kernelements 3, vollständig freigesetzt werden. Wird jedoch während der Entfaltung festgestellt, dass sich die Endoprothese 1 nicht an der korrekten Stelle befindet, so kann diese durch eine Vorwärtsbewegung des rohrförmigen Körpers 2 wieder in den rohrförmigen Körper 2 zusammengefaltet werden. Dies schafft die Voraussetzung, dass die Endoprothese 1 danach entsprechend umpositioniert und an der neuen Position, in der vorgängig beschriebenen Weise, erneut freigesetzt werden kann.

Durch eine Formschlussverbindung zwischen der Endoprothese 1 und dem Kernelement 3, welche durch eine mit der Struktur der inneren Oberfläche der Endoprothese 1 korrespondierende Prägung 11 sowie der in die Prägung 11 eingreifenden Endoprothese 1 erzielt wird, kann sichergestellt werden, dass einerseits die aufzuwendenden Kräfte zum Bewegen des Kernelements 3 relativ zum äusseren Körper 2 gering gehalten werden können, und dass andererseits ein sicheres radiales Ablösen der selbstexpandierenden Endoprothese 1 vom Kernelement 2 auch nach langer Lagerung noch gewährleistet ist. Kurz gesagt, durch eine solche Ausgestaltung der Vorrichtung ist eine sichere und zuverlässige Funktionsweise erreicht. Sie ist auch leicht und ohne grossen Aufwand herzustellen.

Das Verfahren zum Herstellen einer Prägung für eine solche Vorrichtung kann folgendermassen ablaufen:
Eine Endoprothese 1 wird im entfalteten Zustand auf den Bereich B des Kernelements 3 geschoben. Danach wird die Endoprothese 1 im Bereich des Abschnitts 9 des Kernelements 3, in dem keine Prägung auszubilden ist, zusammengefaltet. Danach wird der äussere rohrförmige Körper 2 bis an das proximale Ende des Bereichs 10 des Kernelements 3 vorgeschoben. Dadurch wird die Endoprothese 1 in ihrer Lage fixiert. Anschliessend wird die Endoprothese 1 durch ein an sich bekanntes Presswerkzeug in ihrem freiliegenden Bereich zusammengefaltet und durch das Presswerkzeug an den Bereich 10 des Kernelements 3 gepresst. Nun wird das Presswerkzeug mittel Heissluft erwärmt, so dass die zusammengepresste Endoprothese 1 erhitzt wird und dadurch in das thermoplastisch verformbare Material des Kernelements 3 bzw. in dessen Beschichtung oder Ummantelung 3a gedrückt wird. Nach dem Entfernen des Presswerkzeugs wird der äussere rohrförmige Körper 2 dann soweit vorgeschoben, bis dessen distales Ende an die Rückseite der Spitze 15 des Kernelements 3 zu liegen kommt und die Endoprothese 1 somit vollständig eingeschlossen ist. Das Verfahren läuft entsprechend ab, wenn statt des thermoplastisch verformbaren Materials ein aushärtender Kunststoff eingesetzt wird. Die Schulter 8 muss während der Durchführung dieses Verfahrens nicht unbedingt am proximalen Ende der Endoprothese 1 anliegen. Sie legt sich automatisch an das proximale Ende der Enoprothese 1 an, sobald beim Freisetzen die Endoprothese 1 mit dem Kernelement 3 ausser Eingriff gekommen ist. Die Schulter 8 dient der Endoprothese 1 dann als Widerlager bei der Freisetzung des restlichen, noch im äusseren rohrförmigen Körper 2 eingeschlossenen Endoprothesenteils.

Natürlich sind auch Verfahren denkbar, bei denen immer die gleiche Endoprothese zum Ausbilden der Prägung verwendet wird. Im weiteren ist es auch möglich, die Prägung durch einen am Presswerkzeug ausgebildeten, positiven Abdruck einer Endoprothese anzubringen.

## Patentansprüche

1. Anordnung mit einer selbstexpandierenden Endoprothese (1) und einer Vorrichtung zu deren Implantation, welche Vorrichtung ein inneres langgestrecktes Kernelement (3) und einen äusseren rohrförmigen Körper (2) aufweist, der relativ zum Kernelement (3) verschiebbar ist und dieses in Längsrichtung zumindest teilweise umfasst, wobei zwischen dem Kernelement (3) und dem äusseren Körper (2) die selbstexpandierende Endoprothese (1) zusammengefaltet einschliessbar ist und wobei durch eine Bewegung des rohrförmigen Körpers (2) in Richtung des proximalen Endes des Kernelements (3) die Endoprothese (1) freigegeben und durch eine relative Bewegung des rohrförmigen Körpers (2) in Richtung des distalen Endes des Kernelements (3) die Endoprothese (1) zurückgefaltet wird, dadurch gekennzeichnet, dass das Kernelement (3), zumindest auf einem Teil seiner Oberfläche, ein flächig angebrachtes und mit der Struktur der inneren Oberfläche der Endoprothese (1) korrespondierendes Relief (11) zur Erzielung einer Vielzahl von einzelnen, radial lösbaren Formschlusseingriffsstellen (20) zwischen dem Kernelement (3) und der zumindest teilweise eingeschlossenen Endoprothese (1) aufweist.

2. Anordnung nach Anspruch 1, dadurch gekennzeichnet, dass das Relief (11) individuell der Struktur der inneren Oberfläche der jeweils verwendeten Endoprothese (1) angepasst ist.

3. Anordnung nach Anspruch 1 oder 2, dadurch gekennzeichnet, dass bei zusammengefaltet eingeschlossener Endoprothese (1) die flächig angebrachte Prägung (11) auf dem Kernelement (3) sich über eine Länge von 10-50% der Endoprothese (1) erstreckt.

4. Anordnung nach einem der vorhergehenden Ansprüche, dadurch gekennzeichnet, dass das Kernelement (3) einen prägungsfreien Bereich (9) aufweist, der bei zusammengefaltet eingeschlossener Endoprothese (1) im Bereich des proximalen Endes derselben liegt.

5. Anordnung nach einem der vorhergehenden Ansprüche, dadurch gekennzeichnet, dass das Kernelement (3) zumindest im Bereich seines distalen Endes eine Beschichtung oder Ummantelung (3a) aufweist, in welche die Prägung (11) eingelassen ist.

6. Anordnung nach Anspruch 5, dadurch gekennzeichnet, dass das Kernelement (3) und die gegebenenfalls vorgesehene Beschichtung oder Ummantelung (3a) aus einem thermoplastisch verformbaren Material besteht.

7. Anordnung nach Anspruch 6, dadurch gekennzeichnet, dass als Ummantelung ein Stück Kunststoffschlauch (3a) vorgesehen ist, welches auf das Kernelement (3) aufgeschrumpft ist.

8. Anordnung nach einem der Ansprüche 5 bis 7, dadurch gekennzeichnet, daß die thermoplastische Verformbarkeit des Kernelements (3) geringer als diejenige der Beschichtung bzw. Ummantelung (3a) ist.

9. Anordnung nach einem der vorhergehenden Ansprüche, dadurch gekennzeichnet, dass die Tiefe der Prägung (11) zumindest annähernd 50% der Wandstärke der Endoprothese (1) beträgt.

10. Anordnung nach einem der vorhergehenden Ansprüche, wobei die Endoprothese (1) aus mehreren Lagen eines Halbzeuges besteht, dadurch gekennzeichnet, dass die Endoprothese (1) mit höchstens 50% der Wandstärke des Halbzeugs der innersten Lage mit der Prägung (11) im Eingriff steht.

11. Anordnung nach einem der vorhergehenden Ansprüche, dadurch gekennzeichnet, dass der mittlere Abstand zwischen dem Aussendurchmesser des Kernelements (3) und dem Innendurchmesser des rohrförmigen Körpers (2), im Bereich der Prägung (11), geringer als die Wandstärke der Endoprothese (1) ist.

12. Anordnung nach einem der vorhergehenden Ansprüche, dadurch gekennzeichnet, dass das Kernelement (3) ein Lumen (16) zur Aufnahme eines Führungsdrahtes (17) aufweist.

13. Anordnung nach einem der vorhergehenden Ansprüche, dadurch gekennzeichnet, dass das Kernelement (3) eine proximal zur Endoprothese (1) liegende Schulter (8) aufweist.

14. Anordnung nach Anspruch 13, dadurch gekennzeichnet, dass die Schulter (8) durch einen Ring aus strahlungsopakem Material gebildet ist.

15. Anordnung nach einem der vorhergehenden Ansprüche, dadurch gekennzeichnet, dass das Kernelement (3) im Bereich der Prägung (11) einen grösseren Aussendurchmesser aufweist als die an die Prägung (11) angrenzenden und keine Prägung aufweisenden Bereiche (9, 13).

16. Verfahren zum Herstellen einer Prägung für eine Vorrichtung gemäss den Ansprüchen 1 bis 15, dadurch gekennzeichnet, dass eine Endoprothese (1) im Bereich des distalen Endes des Kernelements (3) um dieses gelegt und in die Oberfläche des Kernelements (3) bzw. in dessen Beschichtung resp. Ummantelung (3a) gedrückt wird.

17. Verfahren zum Herstellen der Prägung gemäss Anspruch 16, dadurch gekennzeichnet, dass die Prägung (11) durch einen Abdruck der Struktur der inneren Oberflächen einer eingeschlossenen Endoprothese (1) geformt wird.

18. Verfahren zum Herstellen der Prägung gemäss Anspruch 16, dadurch gekennzeichnet, dass die Prägung (11) unter Wärmeeinwirkung erzielt wird.

19. Verfahren zum Herstellen der Prägung gemäss Anspruch 16, dadurch gekennzeichnet, dass die Prägung (11) unter Verwendung der individuell in dieser Vorrichtung zum Einsatz kommenden Endoprothese (1) angebracht wird.

20. Verfahren zum Herstellen der Prägung gemäss Anspruch 19, dadurch gekennzeichnet, dass die individuell in dieser Vorrichtung zum Einsatz kommende Endoprothese (1) mit ihrem proximalen Ende in einem Bereich (9), in dem keine Prägung auszubilden ist, zusammengefaltet um das Kernelement (3) gelegt wird, dass der äussere rohrförmige Körper (2) soweit vorgeschoben wird, bis das proximale Ende der Endoprothese (1) soweit zusammengefaltet eingeschlossen ist, dass die Endoprothese (1) in ihrer Lage gegenüber dem äusseren rohrförmigen Körper (2) gesichert ist, dass die Endoprothese (1) in dieser gesicherten Lage ausserhalb des rohrförmigen Körpers (2) zusammengefaltet in die Oberfläche des Kernelements (3) gedrückt wird, und dass danach die Montage der zusammengefaltet eingeschlossenen Endoprothese (3) durch Vorschieben des äusseren rohrförmigen Körpers (2) abgeschlossen wird.

## Claims

1. Arrangement with a self-expanding endoprosthesis (1) and a device for its implantation, said device having an inner elongate core element (3) and an outer tubular body (2) which can be slid in relation to the core element (3) and at least partly encloses this in a lengthwise direction, with it being possible to enclose the folded-up self-expanding endoprosthesis (1) between the core element (3) and the outer body (2) and, by a movement of the tubular body (2) in the direction of the proximal end of the core element (3), the endoprosthesis (1) is released and by a relative movement of the tubular body (2) in the direction of the distal end of the core element (3) the endoprosthesis (1) is folded back, characterized in that the core element (3), at least on a part of its surface, has a flat-spread embossing (11) corresponding to the structure of the internal surface of the endoprosthesis (1) to achieve a multiplicity of individual, radially detachable positive action points (20) between the core element (3) and the at least partly enclosed endoprosthesis (1).

2. Arrangement according to Claim 1, characterized in that the embossing (11) is individually matched to the structure of the internal surface of the particular endoprosthesis (1) used.

3. Arrangement according to Claim 1 or 2, characterized in that, with a folded-up, enclosed endoprosthesis (1), the flat-spread embossing (11) applied on the core element (3) extends over a length of 10-50% of the endoprosthesis (1).

4. Arrangement according to one of the preceding claims, characterized in that the core element (3) has a zone (9) free from embossing which, with a folded-up enclosed endoprosthesis (1), is situated in the zone of the proximal end of the same.

5. Arrangement according to one of the preceding claims, characterized in that the core element (3), at least in the zone of its distal end, has a coating or sheathing (3a), into which the embossing (11) is made.

6. Arrangement according to Claim 5, characterized in that the core element (3) and the coating or sheathing (3a), if provided, is made of a thermoplastically deformable material.

7. Arrangement according to Claim 6, characterized in that, as sheathing, a piece of plastic flexible tube (3a) is provided, which is shrunk onto the core element (3).

8. Arrangement according to one of Claims 5 to 7, characterized in that the thermoplastic deformability of the core element (3) is less than that of the coating or sheathing (3a).

9. Arrangement according to one of the preceding claims, characterized in that the depth of the embossing (11) is at least approximately 50% of the wall thickness of the endoprosthesis (1).

10. Arrangement according to one of the preceding claims, the endoprosthesis (1) consisting of several layers of a semi-finished product, characterized in that the endoprosthesis (1) is engaged with the embossing (11) by not more than 50% of the wall thickness of the semi-finished product of the innermost layer.

11. Arrangement according to one of the preceding claims, characterized in that the average distance between the outside diameter of the core element (3) and the inside diameter of the tubular body (2), in the zone of the embossing (11), is less than the wall thickness of the endoprosthesis (1).

12. Arrangement according to one of the preceding claims, characterized in that the core element (3) has a lumen (16) for holding a guide wire (17).

13. Arrangement according to one of the preceding claims, characterized in that the core element (3) has a shoulder (8) situated proximally to the endoprosthesis (1).

14. Arrangement according to Claim 13, characterized in that the shoulder (8) is formed by a ring of radiation-opaque material.

15. Arrangement according to one of the preceding claims, characterized in that the core element (3) has, in the embossed zone (11), a larger outside diameter than that on the zones (9, 13) adjacent to the embossing (11) and being without embossing.

16. Method for producing an embossing for a device according to Claims 1 to 15, characterized in that an endoprosthesis (1) is placed around the core element (3) in the zone of the distal end thereof and pressed into the surface of the core element (3) or into its coating or sheathing (3a) respectively.

17. Method for producing the embossing according to Claim 16, characterized in that the embossing (11) is moulded by an impression of the structure of the inside surfaces of an enclosed endoprosthesis (1).

18. Method for producing the embossing according to Claim 16, characterized in that the embossing (11) is achieved with the action of heat.

19. Method for producing the embossing according to Claim 16, characterized in that the embossing (11) is applied by using the endoprosthesis (1) to be used individually in this apparatus.

20. Method for producing the embossing according to Claim 19, characterized in that the endoprosthesis (1) to be used individually in this apparatus is placed with its proximal end in a zone (9) in which no embossing is to be produced, folded up around the core element (3), that the outer tubular body (2) is slid forward to such an extent until the proximal end of the endoprosthesis (1) is so far enclosed in a, folded up state, that the endoprosthesis (1) is secured in its position with regard to the outer tubular body (2), that the endoprosthesis (1), in this secured position folded up outside the tubular body (2), is pressed into the surface of the core element (3), and that, thereafter, the assembly of the folded-up, enclosed endoprosthesis (3) is finished by sliding forward the outer tubular body (2).

## Revendications

1. Disposition avec une endoprothèse (1) autoexpansible et un dispositif pour son implantation, lequel dispositif présente un élément central (3) interne et étiré en longueur et un corps (2) externe de forme tubulaire qui peut être déplacé par rapport à l'élément central (3) et comprend au moins partiellement cet élément dans le sens de la longueur, l'endoprothèse (1) autoexpansible pouvant être enfermée entre l'élément central (3) et le corps (2) externe et l'endoprothèse (1) étant libérée par un mouvement du corps (2) tubulaire dans le sens de l'extrémité proximale de l'élément central (3) et étant pliée en reculant par un mouvement relatif du corps (2) tubulaire dans la direction de l'extrémité distale de l'élément central (3), caractérisée en ce que l'élément central (3) présente, au moins sur une partie de sa surface, un relief (11) appliqué en deux dimensions et correspondant à la structure de la surface interne de l'endoprothèse (1) afin d'obtenir une pluralité de points d'engrènement à engagement positif (20) individuels et amovibles dans le sens radial entre l'élément central (3) et l'endoprothèse (1) au moins partiellement enfermée.

2. Disposition selon la revendication, caractérisée en ce que le relief (11) est adapté individuellement à la structure de la surface interne de l'endoprothèse (1) respectivement utilisée.

3. Disposition selon la revendication 1 ou 2, caractérisée en ce que, avec l'endoprothèse (1) repliée et enfermée, l'estampage (11) appliqué en deux dimensions sur l'élément central (3) s'étend sur une longueur représentant 10 à 50 % de l'endoprothèse (1).

4. Disposition selon l'une quelconque des revendications précédentes, caractérisée en ce que l'élément central (3) présente une zone (9) sans estampage qui se situe dans la zone de l'extrémité proximale lorsque l'endoprothèse (1) est repliée et enfermée.

5. Disposition selon l'une quelconque des revendications précédentes, caractérisée en ce que l'élément central (3) présente au moins dans la zone de son extrémité distale un revêtement ou enveloppe (3a) dans lequel ou laquelle l'estampage (11) est encastré.

6. Disposition selon la revendication 5, caractérisée en ce que l'élément central (3) et le revêtement ou l'enveloppe (3a) éventuellement prévu(e) sont à base d'un matériau déformable au plan thermoplastique.

7. Disposition selon la revendication 6, caractérisée en ce qu'on prévoit comme enveloppe un bout de tuyau plastique (3a) qui est emmanché par frettage sur l'élément central (3).

8. Disposition selon l'une quelconque des revendications 5 à 7, caractérisée en ce que la déformabilité thermoplastique de l'élément central (3) est inférieure à celle du revêtement ou de l'enveloppe (3a).

9. Disposition selon l'une quelconque des revendications précédentes, caractérisée en ce que la profondeur de l'estampage (11) est égale au moins approximativement à 50 % de l'épaisseur de paroi de l'endoprothèse (1).

10. Disposition selon l'une quelconque des revendications précédentes, l'endoprothèse (1) comprenant plusieurs couches d'un demi-produit, caractérisée en ce que l'endoprothèse (1) est en prise avec au maximum 50 % de l'épaisseur de paroi du demiproduit de la couche la plus interne avec l'estampage (11).

11. Disposition selon l'une quelconque des revendications précédentes, caractérisée en ce que l'écart moyen entre le diamètre extérieur de l'élément central (3) et le diamètre intérieur du corps (2) de forme tubulaire est inférieur à l'épaisseur de paroi de l'endoprothèse (1) dans la zone de l'estampage (11).

12. Disposition selon l'une quelconque des revendications précédentes, caractérisée en ce que l'élément central (3) présente une cavité (16) pour le logement d'un fil de guidage (17).

13. Disposition selon l'une quelconque des revendications précédentes, caractérisée en ce que l'élément central (3) présente un épaulement (8) situé à proximité de l'endoprothèse (1).

14. Disposition selon la revendication 13, caractérisée en ce que l'épaulement (8) est constitué par une bague à base de matériau opaque au rayonnement.

15. Disposition selon l'une quelconque des revendications précédentes, caractérisée en ce que l'élément central (3) présente dans la zone de l'estampage (11) un diamètre extérieur supérieur aux zones (9, 13) limitrophes de l'estampage (11) et ne présentant pas d'estampage.

16. Procédé pour la fabrication d'un estampage pour un dispositif selon les revendications 1 à 15, caractérisé en ce qu'une endoprothèse (1) est posée dans la zone de l'extrémité distale de l'élément central (3) autour de celui-ci et est appuyée dans la surface de l'élément central (3) et dans son revêtement ou enveloppe (3a).

17. Procédé pour la fabrication de l'estampage selon la revendication 16, caractérisé en ce que l'estampage (11) est formé par une empreinte de la structure des surfaces internes d'une endoprothèse (1) enfermée.

18. Procédé pour la fabrication de l'estampage selon la revendication 16, caractérisé en ce que l'estampage (11) est obtenu par l'effet de la chaleur.

19. Procédé pour la fabrication de l'estampage selon la revendication 16, caractérisé en ce que l'estampage (11) est appliqué en utilisant l'endoprothèse (1) utilisée individuellement dans ce dispositif.

20. Procédé pour la fabrication de l'estampage selon la revendication 19, caractérisé en ce que l'endoprothèse (1) utilisée individuellement dans ce dispositif est posée repliée autour de l'élément central (3) avec son extrémité proximale dans une zone (9) où l'on ne doit pas réaliser d'estampage, en ce que le corps (2) externe de forme tubulaire est avancé jusqu'à ce que l'extrémité proximale de l'endoprothèse (1) soit repliée et enfermée pour que l'endoprothèse (1) soit bloquée dans sa position par rapport au corps (2) externe de forme tubulaire, en ce que l'endoprothèse (1) est repliée dans cette position bloquée à l'extérieur du corps (2) de forme tubulaire et appuyée dans la surface de l'élément central (3) et en ce qu'ensuite le montage de l'endoprothèse (1) repliée et enfermée est terminé par l'avancement du corps (2) extérieur de forme tubulaire.
